(19) Europäisches Patentamt
European Patent Office
Office européen des brevets

(11) Veröffentlichungsnummer: **0 218 553**
**B1**

(12) **EUROPÄISCHE PATENTSCHRIFT**

(45) Veröffentlichungstag der Patentschrift: **18.01.89**

(51) Int. Cl.[4]: **C07C 65/10,** C07C 51/353, B41M 5/22, C07C 65/105, C07C 51/377, B41M 5/12

(21) Anmeldenummer: **86810425.8**

(22) Anmeldetag: **29.09.86**

# (54) Verfahren zur Herstellung von Metallsalzgemischen von ringsubstituierten Salicylsäureverbindungen.

(30) Priorität: **03.10.85 CH 4287/85**

(43) Veröffentlichungstag der Anmeldung: **15.04.87 Patentblatt 87/16**

(45) Bekanntmachung des Hinweises auf die Patenterteilung: **18.01.89 Patentblatt 89/3**

(84) Benannte Vertragsstaaten: **CH DE FR GB LI**

(56) Entgegenhaltungen:
**EP-A- 0 181 283**
**EP-A- 0 194 601**
**GB-A- 2 017 090**

**CHEMICAL ABSTRACTS, Band 95, Nr. 8, August 1981, Seite 46, Spalte 1, Zusammenfassungsnr. 63331v, Columbus, Ohio, US; B.-L. HE et al.: "Friedel-Craft reactions of the chloromethylated styrene-divinylbenzene coploymers", & KAO FEN TZU TUNG HSUN 1980, (6), 328-334namycin" 000**

(73) Patentinhaber: **CIBA-GEIGY AG, Klybeckstrasse 141, CH-4002 Basel(CH)**

(72) Erfinder: **Nachbur, Hermann, Mischelistrasse 21, CH-4153 Reinach(CH)**

**EP 0 218 553 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft ein Verfahren zur Herstellung von Metallsalzgemischen von ringsubstituierten Salicylsäureverbindungen, welche Metallsalze als Farbentwickler für Farbbildner in druckempfindlichen oder wärmeempfindlichen Aufzeichnungsmaterialien Verwendung finden können.

Aus der DE-PS 2 242 250 sind Farbstoffakzeptoren oder Farbentwickler für Farbstoffvorläufer bekannt, die Salze eines mehrwertigen Metalles und einer Salicylsäureverbindung sind, wobei die Salicylsäureverbindung in 3- und 5-Stellung disubstituiert ist. Als derartige Substituenten der Salicylsäure werden beispielsweise α-Methylbenzyl, α,α-Dimethylbenzyl oder Cyclohexyl genannt. Gemäss der DE-PS 2 242 250 werden solche ringsubstituierten Salicylsäureverbindungen mit Hilfe substituierter Phenole und gasförmigem Kohlendioxid hergestellt, wobei die substituierten Phenole durch Umsetzung von Phenolen z.B. mit Styrol, α-Methylstyrol oder Cyclohexylchlorid erhalten werden.

Es wurde nun gefunden, dass man wirtschaftlich günstige Metallsalzgemische von ringsubstituierten Salicylsäureverbindungen erhalten kann, wenn man die Herstellung der Salicylsäureverbindungen durch Umsetzen von Salicylsäure mit einer 1-Phenylethanolverbindung im molaren Verhältnis von 1:2 in Gegenwart eines Halogenids eines mehrwertigen Metalles durchführt.

Gegenstand vorliegender Erfindung ist daher ein Verfahren zur Herstellung eines Gemisches aus einem Metallsalz der Formel

(1) 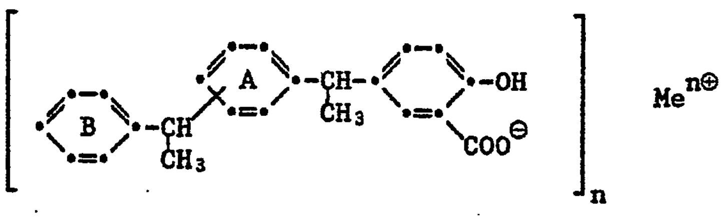

und einem Metallsalz der Formel

(2) 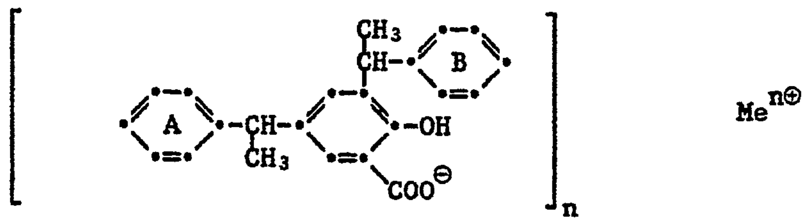

wobei in den Formeln (1) und (2)
Me ein n-wertiges Metallion und
n 2, 3 oder 4 bedeuten und
die Ringe A und B, unabhängig voneinander, unsubstituiert oder durch Halogen, Niederalkyl, Niederalkoxy oder einen α-Methylbenzylrest substituiert sind.

Das Vefahren ist dadurch gekennzeichnet, dass man 2 Mol Salicylsäure mit mindestens 2 Mol eines 1-Phenylethanols der Formel

(3) 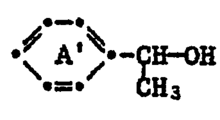

und mindestens 2 Mol eines 1-Phenylethanols der Formel

(4) 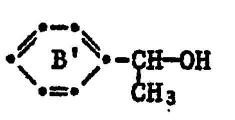 ,

worin die Benzolringe A' und B' unsubstituiert oder durch Halogen, Niederalkyl oder Niederalkoxy substituiert sind, in Gegenwart eines Halogenides eines mehrwertigen Metalles mit einem Atomgewicht von

26 bis 66, insbesondere von Aluminium, Zink, Vanadium, Chrom, Mangan, Eisen, Kobalt, Nickel oder Kupfer umsetzt und 2n Mol des erhaltenen Gemisches aus der Salicylsäureverbindung der Formel

(5) 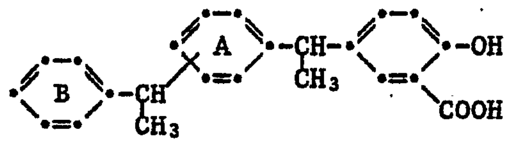

und der Salicylsäureverbindung der Formel

(6) 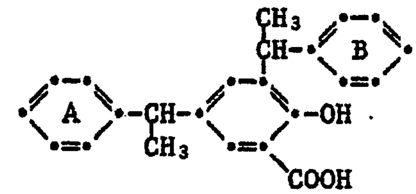

wobei in den Formeln (5) und (6) A und B die für Formeln (1) und (2) angegebenen Bedeutungen haben, mit 2 Mol des Salzes eines n-wertigen Metalls einer anorganischen Säure oder einer niederen aliphatischen Carbonsäure weiter umsetzt, wobei n die angegebene Bedeutung hat.

Die erfindungsgemäss hergestellten Metallsalzgemische können in einem Gewichtsverhältnis von 9:1 bis 1:9 vorliegen.

Die bei den Metallsalzen der Formeln (1) und (2) zweifach bis vierfach vorkommenden Salicylatbestandteile können gleich oder verschieden sein. Vorzugsweise sind sie jeweils identisch.

Niederalkyl und Niederalkoxy stellen in der Regel Gruppen dar, die 1 bis 5, insbesondere 1 bis 3 Kohlenstoffatome aufweisen, wie z.B. Methyl, Ethyl, Isopropyl, sek.-Butyl, tert.-Butyl, Amyl oder Isoamyl bzw. Methoxy, Ethoxy, Isopropoxy, n-Butoxy oder tert.-Butoxy.

Halogen bedeutet beispielsweise Fluor, Jod, Brom oder vorzugsweise Chlor.

Die erfindungsgemäss hergestellten Metallsalze leiten sich vorteilhafterweise von 2-, 3- oder 4wertigen Metallen mit einem Atomgewicht von 24 bis 210, vorzugsweise 26 bis 120 ab. Beispiele derartiger Metalle sind Aluminium, Barium, Blei, Cadmium, Calcium, Chrom, Eisen, Gallium, Kobalt, Kupfer, Magnesium, Mangan, Molybdän, Nickel, Quecksilber, Silber, Strontium, Tantal, Titan, Vanadium, Wolfram, Zink, Zinn und Zirkonium. Dabei sind Aluminium, Vanadium, Zinn, Zirkonium und insbesondere Zink bevorzugt.

Die

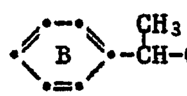-Gruppe

der Verbindungen der Formeln (1) und (5) befindet sich vorteilhafterweise in p-Stellung zur Ethylidengruppe.

Die Ringe A und B sind vorzugsweise nicht weiter substituiert. Falls die Ringe A und B Substituenten aufweisen, sind sie in erster Linie durch Halogen, Methyl, Methoxy oder α-Methylbenzyl weiter substituiert. Pro Benzolring A oder B können vorteilhafterweise 1 oder 2 Substituenten vorhanden sein. Der α-Methylbenzylrest ist in der Regel im Benzolring B vorhanden.

Praktisch wichtige Metallsalzgemische, die erfindungsgemäss hergestellt und vorteilhaft als Farbentwickler verwendet werden, sind Aluminiumsalze oder noch bevorzugter Zinksalze der substituierten Salicylsäureverbindungen der Formeln (5) und (6), in denen der entsprechende Ring B unsubstituiert ist.

Im Vordergrund des Interesses steht als Farbentwickler das Gemisch aus dem Zinksalz der 5-[α-Methyl-4'-(α-methylbenzyl)-benzyl-]salicylsäure und dem Zinksalz der 3,5-Bis-[α-methylbenzyl-]salicylsäure.

Die Herstellung des Gemisches aus den freien Salicylsäureverbindungen der Formeln (5) und (6) erfolgt vorteilhafterweise bei einer Temperatur zwischen 20°C und der Rückflusstemperatur, vorzugsweise bei 80°C bis 150°C. Gewünschtenfalls kann ein organisches Lösungsmittel verwendet werden.

Die Reaktionsdauer hängt in der Regel von der Temperatur des Reaktionsmediums ab und liegt zweckmässigerweise zwischen ½ und 5 Stunden, vorzugsweise 1 bis 3 Stunden.

Vorzugsweise sind die 1-Phenylethanolkomponenten der Formeln (3) und (4) identisch.

Als Beispiele für geeignete 1-Phenylethanolkomponenten der Formeln (3) und (4) seien genannt: 1-Phenylethanol, 1-Tolylethanol, 1-Xylylethanol oder 1-(Chlorphenyl)-ethanol.

Als Halogenid, das als Katalysator dient, kann die Fluorid, Iodid, Bromid oder vorzugsweise ein Chlorid zowie auch ein Pseudohalogenid wie ein Rhodanid in Betracht kommen. Bevorzugter Katalysator ist Zinkchlorid. Der Anteil an Halogenid als Katalysator liegt vorteilhafterweise bei 10 bis 50 Mol%, vorzugsweise 15 bis 30 Mol%, bezogen auf Salicylsäure.

Geeignete organische Lösungsmittel, die das Reaktionsmedium bilden können, sind cycloaliphatische oder vorzugsweise aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Chlorbenzol, Chlortoluol oder Dichlorbenzol; cyclische Ether, wie z.B. Dioxan oder Tetrahydrofuran; Dimethylsulfoxid oder Nitrile aliphatischer Monocarbonsäuren, wie z.B. Acetonitril, Propionitril oder Butyronitril. Auch Mischungen der genannten Lösungsmittel können verwendet werden. Bevorzugte Lösungsmittel sind Chlorbenzol, Chlortoluol und besonders Toluol.

Das erhaltene Gemisch von den freien Salicylsäureverbindungen kann direkt zur Herstellung der Metallsalze der Formeln (1) und (2) weiterverwendet werden.

Falls eine Isolierung der substituierten Salicylsäureverbindungen der Formeln (5) und (6) erwünscht ist, wird z.B. die saure Lösung des Umsetzungsproduktes zuerst mit einer wässerigen Natriumhydroxydlösung neutralisiert und dann die neutrale Lösung mit einer niederen Carbonsäure oder einer anorganischen Säure angesäuert, worauf das Produkt in Form eines Öls ausfällt und abgetrennt wird. Danach können die einzelnen Salicylsäureverbindungen chromatographisch voneinander getrennt werden.

Die Metallisierung des Salicylsäuregemisches erfolgt vorteilhafterweise in einer alkalischen Lösung der verwendeten Salicylsäureverbindungen und vorzugsweise in Gegenwart einer Alkaliverbindung, wie z.B. Alkalimetallhydroxide, -carbonate oder -bicarbonate oder Ammoniumhydroxid, Ammoniumcarbonat oder Ammoniumhydrogencarbonat.

Die Metallisierung kann schon bei einer Temperatur von 5 bis 25°C vorgenommen werden. In gewissen Fällen und besonders bei Verwendung von organischen Aluminiumsalzen ist es nötig bei höheren Temperaturen, vorzugsweise 70 bis 200°C zu arbeiten.

Man kann die Reaktionspartner aber auch in einer Schmelze umsetzen. Als Schmelzmittel eignen sich z.B. Salze niederer Fettsäuren, wie z.B. Natriumacetat, Amide niederer Fettsäuren, wie z.B. Acetamid, ferner Harnstoff oder Thioharnstoff oder deren N-Substitutionsprodukte.

Als metallabgebende Mittel verwendet man zweckmässig die Metallsalze von Mineralsäuren oder Carbonsäuren mit 1 bis 6 Kohlenstoffatomen, insbesondere Sulfate, Halogenide (Chloride), Nitrate, Formiate, Acetate, Propionate, Oxalate oder Citrate.

Als Beispiele für anorganische Metallsalze seien die Zinksalze Zinkchlorid, Zinksulfat oder Zinknitrat sowie auch Aluminiumsulfat, Zinndichlorid oder Zirkonoxychlorid genannt.

Organische Metallsalze sind z.B. Zinkdiacetat, Zinkoxalat, Aluminiumtriisopropylat oder Aluminium-sek.butylat.

Anstelle der genannten Zinksalze können auch Zinkoxid oder Zinkcarbonat verwendet werden, wobei in diesem Falle die Umsetzung mit dem Salicylsäuregemisch, vorzugsweise in Gegenwart von Ammoniumformiat durchgeführt wird.

Eine besonders zweckmässige Ausführungsform zur Herstellung der Metall-Verbindungen der Formeln (1) und (2), in denen Me das Zinkion ist, besteht darin, dass man ein Reaktionsmedium bestehend aus etwa 1 Mol, vorzugsweise 1,0 bis 1,2 Mol Salicylsäure, 2 bis 3 Mol 1-Phenylethanol und 0,2 bis 0,3 Mol Zinkchlorid auf eine Temperatur von 80 bis 170°C vorzugsweise 100–150°C erhitzt und während 1 bis 3 Stunden kondensiert. Darauf wird wässerige Natriumhydroxidlösung zugegeben und die alkalische Lösung mit einem anorganischen Zinksalz, vorzugsweise Zinkchlorid behandelt, worauf das erhaltene Gemisch aus den Zinksalzen der entsprechenden Salicylsäureverbindungen isoliert wird.

Die erfindungsgemäss hergestellten Metallsalzgemische sind praktisch farb- und geruchlos und eignen sich vor allem als Farbentwickler für Farbbildner. Sie sind mit den üblichen Farbbildnern sehr reaktiv, so dass damit spontane, beständige und nicht verblassende Aufzeichnungen oder Kopien erhalten werden können. Die erfindungsgemäss hergestellten Metallsalzgemische werden vorzugsweise als Farbentwickler für Aufzeichnungsmaterialien verwendet, die sowohl Kopier- als auch Registriermaterialien sein können. Das Aufzeichnungsmaterial kann druckempfindlich oder wärmeempfindlich sein.

Die im Aufzeichnungsmaterial in Betracht kommenden Farbbildner sind bekannte farblose oder schwach gefärbte Stoffe, die, sofern sie mit dem Gemisch aus den Metallsalzen der Formeln (1) und (2) in Kontakt kommen, farbig werden oder die Farbe ändern. Farbbildner oder deren Mischungen können verwendet werden, die z.B. den Klassen der Phthalide, Azaphthalide, Fluorane, Spiropyrane, Spirodipyrane, Azomethine, Chinazoline, Leukoauramine, Triarylmethan-leukofarbstoffe, Carbazolylmethane, Rhodaminlaktame, Chromenopyrazole, Phenoxazine, Phenothiazine, sowie der Chromeno- oder Chromanfarbbildner angehören. Als Beispiele solcher geeigneten Farbbildner seien genannt Kristallviolettlacton, 3,3-(Bisaminophenyl)-phthalide, 3,3-(Bis-substituierte-indolyl)-phthalide, 3-(Aminophenyl)-3-indolyl-phthalide, 3-(Aminophenyl-3-indolylazaphthalide, 6-Dialkylamino-2-n-octyl-amino-fluorane, 6-Dialkylamino-2-arylamino-fluorane, z.B. 6-Diethylamino-2-(2'-chlorphenylamino)-fluoran, 6-Dibutylamino-2-(2'-chlorphenylamino)-fluoran, 6-Dialkylamino-3-methyl-2-arylamino-fluorane, z.B. 2-Anilino-3-methyl-6-diethylaminofluoran oder 2-(2',4'-Dimethylanilino)-3-methyl-6-diethylaminofluoran, 6-Dialkylamino-2- oder -3-niederalkyl-fluorane, 6-Dialkylamino-2-dibenzylamino-fluorane, 6-Pyrrolidino-2-dibenzylaminofluoran, 6-N-Cyclohexyl-N-niederalkylamino-3-methyl-2-arylamino-fluorane, 6-Pyrrolidino-2-arylamino-fluorane, Bis-(aminophenyl)-furyl- oder -phenyl- oder -carbazolyl-methane, 3'-Phenyl-7-dialkylamino-2,2'-spirodibenzopyrane, Bis-dialkylamino-benzhydrolalkyl- oder -aryl-sulfinate, Benzoyldialkylamino-phenothiazine oder -phenoxazine.

In den folgenden Herstellungsbeispielen und Anwendungsbeispielen beziehen sich die Prozentsätze, wenn nichts anderes angegeben ist, auf das Gewicht und Teile sind Gewichtsteile.

Herstellungsbeispiele

<u>Beispiel 1:</u> 465 g Salicylsäure, 735 g 1-Phenylethanol und 36 g Zinkchlorid (wasserfrei) werden unter Rühren während 3 Stunden bei 120°C kondensiert. Das gebildete Wasser wird laufend abdestilliert, wobei nach Ablauf von 3 Stunden noch während 30 Minuten bei reduziertem Druck (300–400 Torr) destilliert wird. Danach wird auf 90°C gekühlt und 1944 ml einer wässerigen 2n-Natriumhydroxidlösung zugegeben. Die erhaltene Lösung lässt man einige Stunden bei Raumtemperatur stehen, worauf das als untere Phase abgeschiedene zähflüssige Öl abgetrennt wird. Hierauf lässt man die wässrige Lösung unter Rühren bei 10–15°C zu einer Lösung von 318 g Zinkchlorid in 6 Liter Wasser in dünnem Strahl zufliessen. Das ausgeschiedene Zinksalz wird abfiltriert, abgepresst, in 5 Liter Wasser aufgenommen, wieder abfiltriert, abgepresst und bei 50–70°C im Vakuum getrocknet. Man erhält 928 g eines pulverförmigen Produktes, das ein Gemisch aus dem Zinksalz der Formel

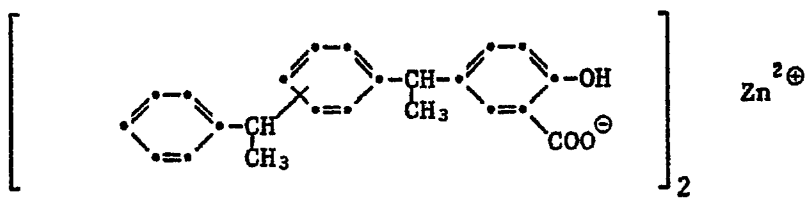

(11)

und dem Zinksalz der Formel

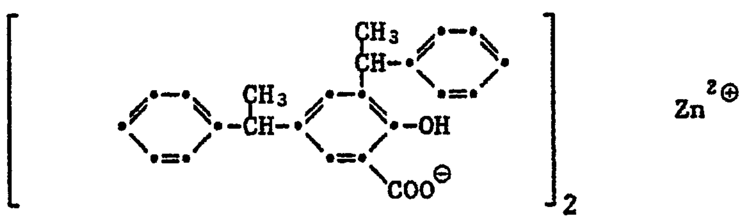

(12)

darstellt. Das NMR-Spektrum dieses Gemisches zeigt ein Gewichtsverhältnis von 3:2.

Das Gemisch schmilzt bei 90–130°C.

<u>Beispiel 2:</u> 77,5 g Salicylsäure, 140 g 1-p-Tolylethanol (97%) und 6 g Zinkchlorid (wasserfrei) werden unter Rühren während 3 Stunden bei 120°C kondensiert. Das gebildete Wasser wird laufend abdestilliert, wobei nach Ablauf von 3 Stunden noch während 15 Minuten unter Vakuum destilliert wird. Danach wird auf 90°C abgekühlt und 295 ml einer wässrigen 2n-Natriumhydroxydlösung zugegeben. Die erhaltene Lösung lässt man einige Stunden bei Raumtemperatur stehen, worauf das als untere Phase abgeschiedene zähflüssige Öl abgetrennt wird. Hierauf lässt man die wässrige Lösung unter Rühren bei 10–15°C zu einer Lösung von 50 g Zinkchlorid in 400 ml Wasser in dünnem Strahl zufliessen. Das ausgeschiedene Zinksalz wird mit 500 ml Wasser verdünnt, abfiltriert, abgepresst, in 300 ml Wasser aufgeschlemmt, wieder abfiltriert und bei 50–70°C im Vakuum getrocknet. Man erhält 156,6 g eines beigefarbigen pulverförmigen Produktes, das ein Gemisch aus dem Zinksalz der Formel

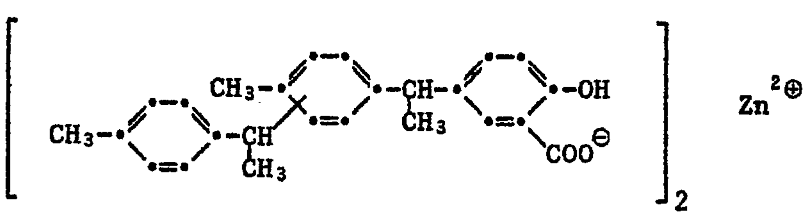

(13)

und dem Zinksalz der Formel

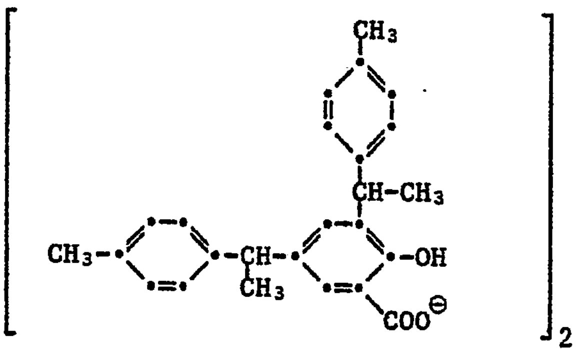

Zn$^{2\oplus}$ (14)

darstellt. Das $^1H$ NMR-Spektrum dieses Gemisches zeigt ein Gewichtsverhältnis von 3:2.
Das Gemisch schmilzt bei ~120–150°C.

Beispiel 3: a) 77,5 g Salicylsäure, 125 g 1-Phenylethanol und 6 g Zinkchlorid (wasserfrei) werden unter Rühren während 3 Stunden bei 120°C kondensiert. Das gebildete Wasser wird laufend abdestilliert, wobei nach Ablauf von 3 Stunden noch während 15 Minuten unter Vakuum destilliert wird. Danach wird auf 90°C abgekühlt und 324 ml einer wässrigen 2n-Natriumhydroxydlösung zugegeben. Die erhaltene Lösung wird einige Stunden bei Raumtemperatur stehen gelassen, worauf das als untere Phase abgeschiedene Öl abgetrennt wird. Man erhält 546 g einer braunen, klaren Lösung, welche einen Gehalt von 35 Gew.% eines ca. 3:2 Gemisches des Na-Salzes der Salicylsäureverbindungen der Formeln (15) und (16) enthält.

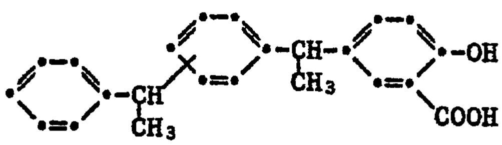

(15)

und

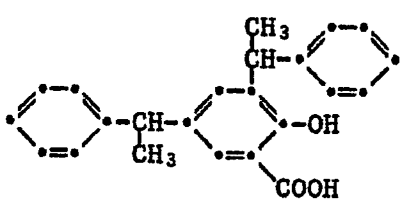

(16)

b) Zu einer Lösung von 15,7 g Zirkonoxychlorid $ZrOCl_2 \cdot 6H_2O$ g in 150 g Wasser lässt man bei 20°C unter Rühren 105 g der gemäss a) hergestellten Na-Salzlösung zutropfen. Das ausgefällte Zirkonsalz wird mit weiteren 250 g Wasser verdünnt, wodurch die Ausfällung praktisch vollständig wird. Das Produkt wird abfiltriert, nochmals in 250 g Wasser aufgeschlämmt, wieder abfiltriert und in feuchtem Zustand in 100 ml Toluol suspendiert. Durch azeotrope Entwässerung unter Vakuum bei anfangs 60°, später bei 95°C erhält man das Zirkonsalz mit einem $H_2O$-Gehalt von 0,8%.

Ausbeute: 37 g (ca. 92,5% d.Th.) graues Pulver; FP ~100–140°C
Analyse: Ber. Zr = 11,43% Gef. Zr. = 10,9%
Das Produkt ist das Zirkonsalz eines 3:2-Gemisches der Salicylsäureverbindungen der Formeln (15) und (16).

Beispiel 4: 105 g der gemäss Beispiel 3a) hergestellten Na-Salzlösung werden im Vakuum bei 60°C zur Trockene eingedampft. Das entwässerte Salz wird bei 20°C in 100 ml Methanol gelöst. Diese Lösung lässt man bei 20°C zu einer Lösung von 10,65 g Zinndichlorid in 100 ml Methanol unter Rühren zutropfen. Da nur eine teilweise Ausfällung des Zinnsalzes eintritt, wird das Methanol bei 60°C im Vakuum entfernt. Der Rückstand wird in 100 ml Toluol bei 20°C gelöst und von unlöslichen Anteilen abfiltriert. Nach dem Entfernen des Toluols bei 95°C unter Vakuum erhält man 37,5 g (92,7% d.Th.) Zinnsalz, welches bei 0°C ein beigefarbiges Pulver ist, und >30°C in einem halbfesten Zustand vorliegt (Fp ca. 20–60°C).
Analyse: Ber. Sn = 14,66%; Gef. Sn = 16,6%

Das Produkt ist das Zinnsalz eines ca. 3:2-Gemisches der Salicylsäureverbindungen der Formeln (15) und (16).

Beispiel 5: Zu einer Lösung von 13,3 g Aluminiumsulfat $Al_2(SO_4)_3 \cdot 18H_2O$ in 100 g Wasser lässt man bei 20°C unter Rühren 105 g der gemäss Beispiel 3a) hergestellten Na-Salzlösung zutropfen. Man erhält eine deutliche Fällung des Al-Salzes, welches abfiltriert, nochmals in 100 g Wasser aufgeschlämmt und wieder abfiltriert wird. Das feuchte Produkt wird in 250 ml Toluol gelöst. Durch azeotrope Entwässerung unter Vakuum bei 95°C erhält man das Al-Salz mit einem $H_2O$-Gehalt von 2,2%.

Ausbeute: 32 g (ca. 88% d.Th. ber. auf wasserfreies Produkt) beigefarbiges Pulver; Fp ~80–120°C.

Analyse: Ber. Al = 2,54%; Gef. Al = 2,37%

Das Produkt ist das Aluminiumsalz eines 3:2-Gemisches der Salicylsäureverbindungen der Formeln (15) und (16).

Anwendungsbeispiele

Beispiel 1 (Druckempfindliches Aufzeichnungssystem): Eine feingemahlene wässrige Dispersion (2–4 µm), die einen 38%igen Feststoffgehalt bestehend aus
1 Teil des Zinksalzgemisches gemäss Herstellungsbeispiel 1,
7,4 Teilen China Clay,
0,8 Teilen eines Kondensationsproduktes aus Naphthalinsulfonsäure und Formaldehyd und
0,9 Teilen eines Styrol/Butadiencopolymerisates (100%)
aufweist, wird auf ein Streichrohpapier mit einem Flächengewicht von 48 g/m² mit einem Rakel aufgetragen. Auftragsgewicht trocken: 6–7 g/m².

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 2: Eine feingemahlene wässrige Dispersion (2–4 µm), die einen 38%igen Feststoffgehalt bestehend aus
1 Teil des Zinksalzgemisches gemäss Herstellungsbeispiel 2
10 Teilen China Clay und
0,6 Teilen Polyvinylalkohol
aufweist, wird auf ein Streichrohpapier mit einem Flächengewicht von 48 g/m² mit einem Rakel aufgetragen. Auftragsgewicht trocken: 5–6 g/m².

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 3: Eine feingemahlene wässrige Dispersion (2–4 µm), die einen 38%igen Feststoffgehalt bestehend aus
1 Teil des Zirkonsalzgemisches gemäss Herstellungsbeispiel 3
10 Teilen China Clay und
0,6 Teilen Polyvinylalkohol
aufweist, wird auf ein Streichrohpapier mit einem Flächengewicht von 48 g/m² mit einem Rakel aufgetragen. Auftragsgewicht trocken: 5–6 g/m².

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 4: Eine feingemahlene wässrige Dispersion (2–4 µm), die einen 38%igen Feststoffgehalt bestehend aus
1 Teil des Aluminiumsalzgemisches gemäss Herstellungsbeispiel 5
10 Teilen China Clay und
0,6 Teilen Polyvinylalkohol
aufweist, wird auf ein Streichrohpapier mit einem Flächengewicht von 48 g/m² mit einem Rakel aufgetragen. Auftragsgewicht trocken: 5–6 g/m².

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 5: 0,6 Teile des Zinnsalzgemisches gemäss Herstellungsbeispiel 4 werden in 8 Teilen Toluol gelöst und auf einem Streichrohpapier von 1 $m^2$ Fläche (Gewicht: 48 g/$m^2$) aufgetragen. Auftragsgewicht trocken: ca. 0,6 g/$m^2$.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

**Patentansprüche**

1. Verfahren zur Herstellung eines Metallsalzgemisches von einem Metallsalz der Formel

(1) 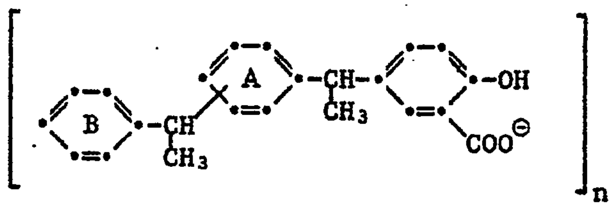 $Me^{n\oplus}$

und einem Metallsalz der Formel

(2) 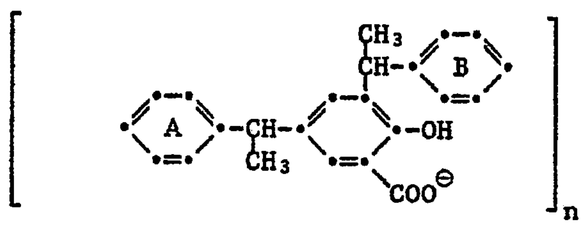 $Me^{n\oplus}$

wobei in den Formeln (1) und (2)
Me ein n-wertiges Metallion und
n 2, 3 oder 4 bedeuten und
die Ringe A und B, unabhängig voneinander, unsubstituiert oder durch Halogen, Niederalkyl, Niederalkoxy oder einen α-Methylbenzylrest substituiert sind, dadurch gekennzeichnet, dass man 2 Mol Salicylsäure mit mindestens 2 Mol eines 1-Phenylethanols der Formel

(3) 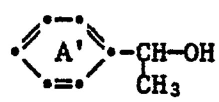

und mindestens 2 Mol eines 1-Phenylethanols der Formel

(4) 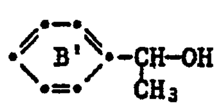 ,

worin die Benzolringe A' und B' unsubstituiert oder durch Halogen, Niederalkyl oder Niederalkoxy substituiert sind, in Gegenwart eines Halogenids eines mehrwertigen Metalles mit einem Atomgewicht von 26 bis 66 umsetzt und 2n Mol des erhaltenen Gemisches aus der Salicylsäureverbindung der Formel

(5) 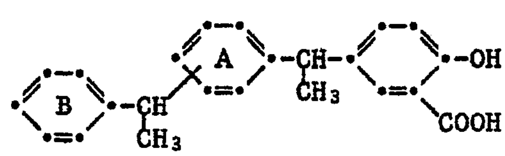

und der Salicylsäureverbindung der Formel

(6) 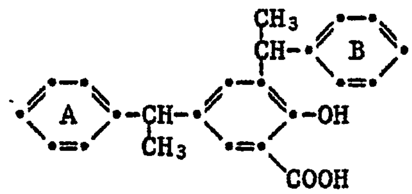

wobei in den Formeln (5) und (6) A und B die für Formeln (1) und (2) angegebenen Bedeutungen haben, mit 2 Mol des Salzes eines n-wertigen Metalls einer anorganischen Säure oder einer niederen aliphatischen Carbonsäure weiter umsetzt, wobei n die angegebene Bedeutung hat.

2. Verfahren gemäss Anspruch 1, dadurch gekennzeichnet, dass in den Formeln (1) und (2) Me das Aluminium-, Zirkonium-, Vanadium-, Zinn- oder vorzugsweise Zinkion ist.

3. Verfahren gemäss einem der Ansprüche 1 und 2, dadurch gekennzeichnet, dass in den Formeln (1) und (2) der Ring B unsubstituiert oder durch α-Methylbenzyl substituiert ist.

4. Verfahren gemäss einem der Ansprüche 1 und 2, dadurch gekennzeichnet, dass in den Formeln (1) und (2) die Ringe A und B unsubstituiert sind.

5. Verfahren gemäss einem der Ansprüche 1 bis 4, dadurch gekennzeichnet, dass in den Formeln (1) und (2) Me das Aluminium oder vor allem das Zinkion ist und der Ring B unsubstituiert ist.

6. Verfahren gemäss einem der Ansprüche 1 bis 5, dadurch gekennzeichnet, dass das Verfahrenserzeugnis ein Gemisch von dem Zinksalz der 5-[α-Methyl-4'-(α-methylbenzyl)-benzyl-]salicylsäure und dem Zinksalz der 3,5-Bis(α-methylbenzyl-)salicylsäure ist.

7. Verfahren gemäss einem der Ansprüche 1 bis 6, dadurch gekennzeichnet, dass man ein Gemisch der Metallsalze der Formeln (1) und (2) herstellt, wobei das Gewichtsverhältnis der Metallsalzkomponenten 1:9 bis 9:1 beträgt.

8. Verfahren gemäss einem der Ansprüche 1 bis 7, dadurch gekennzeichnet, dass man die Umsetzung der Salicylsäure mit den 1-Phenylethanolkomponenten der Formeln (3) und (4) in Gegenwart eines Halogenides von Chrom, Eisen, Kobalt, Kupfer, Mangan, Nickel, Aluminium, Vanadium oder vorzugsweise Zink durchführt.

**Claims**

1. A process for the preparation of a metal salt mixture of a metal salt of formula

(1) 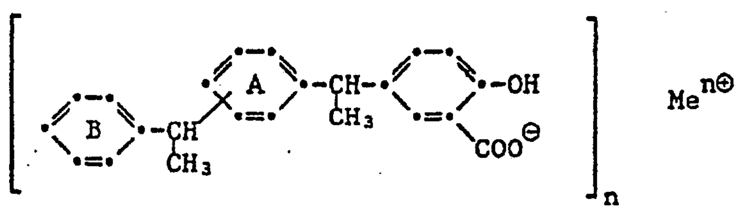

and a metal salt of a formula

(2) 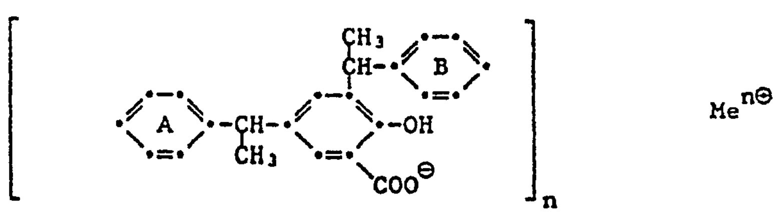

in which formulae (1) and (2)
Me is an n-valent metal ion,
n is 2, 3 or 4, and
each of the rings A and B independently of the other is unsubstituted or substituted by halogen, lower alkyl, lower alkoxy or an α-methylbenzyl radical, which process comprises reacting 2 moles of salicylic acid with at least 2 moles of a 1-phenylethanol of formula

(3) 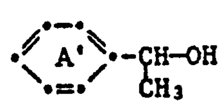

and at least 2 moles of a 1-phenylethanol of formula

(4) 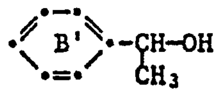 ,

in which the benzene rings A' and B' are unsubstituted or substituted by halogen, lower alkyl or lower alkoxy, in the presence of a halide of a polyvalent metal having an atomic weight from 26 to 66, and, in a further step, reacting 2n moles of the resultant mixture of the salicylic acid compound of formula

(5) 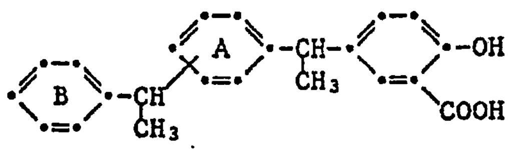

and the salicylic acid compound of formula

(6) 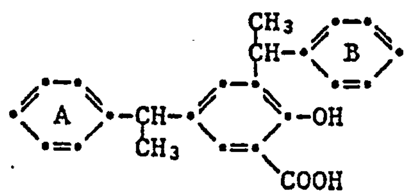

in which formulae (5) and (6) A and B are as defined from formulae (1) and (2), with 2 moles of the salt of an n-valent metal of an inorganic acid or of a lower aliphatic carboxylic acid, where n has the given meaning.

2. A process according to claim 1, wherein Me in formulae (1) and (2) is the aluminium, zirconium, vanadium, tin or preferably the zinc ion.

3. A process according to either of claims 1 and 2, wherein the ring B in formulae (1) and (2) is unsubstituted by a-methylbenzyl.

4. A process according to either of claims 1 and 2, wherein the rings A and B in formulae (1) and (2) are unsubstituted.

5. A process according to any one of claims 1 to 4, wherein Me in formulae (1) and (2) is the aluminium ion or in particular the zinc ion and the ring B is unsubstituted.

6. A process according to any one of claims 1 to 5, wherein the product of the process is a mixture of the zinc salt of 5-(α-methyl-4'-(α-methylbenzyl)benzyl)-salicylic acid and the zinc salt of 3,5-bis (α-methylbenzyl)-salicylic acid.

7. A process according to any one of claims 1 to 6, which comprises preparing a mixture of the metal salts of formulae (1) and (2), the metal salt components being in the weight ratio of 1:9 to 9:1.

8. A process according to any one of claims 1 to 7, wherein the reaction of salicylic acid with the 1-phenylethanol components of formulae (3) and (4) is carried out in the presence of a halide of chromium, iron, cobalt, copper, manganese, nickel, aluminium, vanadium or preferably zinc.

## Revendications

1. Procédé de préparation d'un mélange de sels métalliques consistant en un sel métallique de formule

(1) 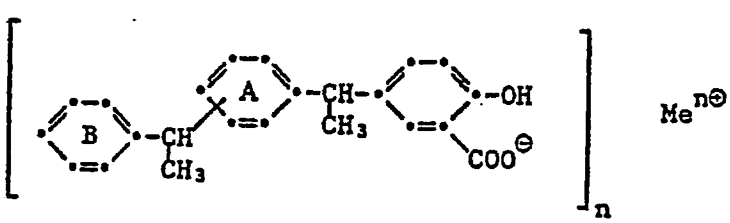

et un sel métallique de formule

(2) 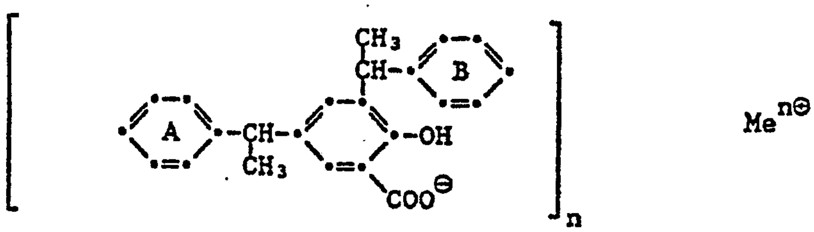

dans lesquelles formules (1) et (2)
Me représente un ion métallique de valence n, et
n est égal à 2, 3 ou 4,
et les cycles A et B, indépendamment l'un de l'autre, sont non substitués ou substitués par des halogènes, des groupes alkyle inférieurs, alcoxy inférieurs ou un groupe alphaméthylbenzyle, caractérisé en ce que l'on fait réagir 2 moles d'acide salicylique avec au moins 2 moles d'un 1-phényléthanol de formule

(3) 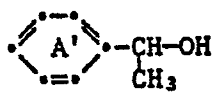

et au moins 2 moles d'un 1-phényléthanol de formule

(4) 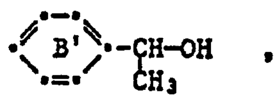

dans lesquelles les cycles benzéniques A' et B' sont non substitués ou substitués par des halogènes ou des groupes alkyle inférieurs ou alcoxy inférieurs, en présence d'un halogénure d'un métal polyvalent de poids atomique 22 à 66, puis on fait réagir 2n moles du mélange obtenu, consistant en le dérivé de l'acide salicylique de formule

(5) 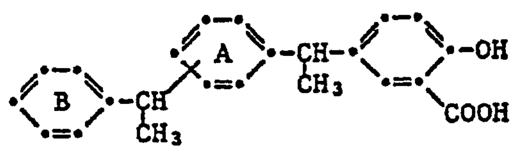

et le dérivé de l'acide salicylique de formule

(6) 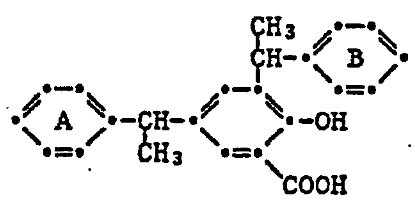

dans lesquelles formules (5) et (6) A et B ont les significations indiquées à propos des formules (1) et (2), avec 2 moles du sel d'un métal de valence n d'un acide minéral ou d'un acide carboxylique aliphatique inférieur, n ayant les significations indiquées ci-dessus.

2. Procédé selon la revendication 1, caractérisé en ce que, dans les formules (1) et (2), Me représente l'ion aluminium, zirconium, vanadium, étain ou de préférence zinc.

3. Procédé selon l'une des revendications 1 et 2, caractérisé en ce que, dans les formules (1) et (2), le cycle B est non-substitué ou substitué par un groupe alphaméthylbenzyle.

4. Procédé selon la revendication 1 et 2, caractérisé en ce que, dans les formules (1) et (2), les cycles A et B sont non-substitués.

5. Procédé selon l'une des revendications 1 à 4, caractérisé en ce que, dans les formules (1) et (2), Me représente l'ion aluminium ou, surtout, l'ion zinc, et le cycle B est non-substitué.

6. Procédé selon l'une des revendications 1 à 5, caractérisé en ce que le produit préparé est un mélange du sel de zinc de l'acide 5-[alpha-méthyl-4'-(alpha-méthylbenzyl)-benzyl]-salicylique et du sel de zinc de l'acide 3,5-bis-(alpha-méthylbenzyl)-salicylique.

7. Procédé selon l'une des revendications 1 à 6, caractérisé en ce que l'on prépare un mélange des sels métalliques de formules (1) et (2) où les proportions relatives en poids entre les sels métalliques composants sont de 1:9 à 9:1.

8. Procédé selon l'une des revendications 1 à 7, caractérisé en ce que la réaction entre l'acide salicylique et les composants 1-phényléthanols de formules (3) et (4) est effectuée en présence d'un halogénure du chrome, du fer, du cobalt, du cuivre, du manganèse, du nickel, de l'aluminium, du vanadium ou, de préférence, du zinc.